**Europäisches Patentamt**

**European Patent Office**

**Office européen des brevets**

(19)

(11) Publication number : **0 105 096 B2**

(12)

# NEW EUROPEAN PATENT SPECIFICATION

(45) Date of publication of the new patent specification : **25.03.92 Bulletin 92/13**

(51) Int. Cl.⁵ : **A61M 1/14**

(21) Application number : **83106506.5**

(22) Date of filing : **04.07.83**

(54) **A tube set intended for extracorporeal treatment of blood and similar perishable liquids.**

(30) Priority : **10.09.82 SE 8205159**

(43) Date of publication of application :
**11.04.84 Bulletin 84/15**

(45) Publication of the grant of the patent :
**29.07.87 Bulletin 87/31**

(45) Mention of the opposition decision :
**25.03.92 Bulletin 92/13**

(84) Designated Contracting States :
**AT BE CH DE FR GB IT LI LU NL**

(56) References cited :
**EP-A- 0 033 080**
**EP-A- 0 103 073**
**DE-A- 2 737 922**
**DE-A- 2 754 809**
**DE-B- 2 740 062**
**FR-A- 2 459 663**
**GB-A- 2 021 418**
**US-A- 3 550 619**

(73) Proprietor : **Gambro Lundia AB**
**Box 10101**
**S-220 10 Lund (SE)**

(72) Inventor : **Carlsson, Per-Olov Arne Vilhelm**
**Morkullevägen 11**
**S-280 10 Sösdala (SE)**
Inventor : **Falkvall, Thore Edvard**
**Karl X Gustafs gata 67**
**S-252 40 Helsingborg (SE)**
Inventor : **Karlberg, Rolf Emil**
**Ympningsvägen 32**
**S-240 10 Veberöd (SE)**

(74) Representative : **Boberg, Nils Gunnar Erik**
**Gambro AB Patent Department Box 10101**
**S-220 10 Lund (SE)**

EP 0 105 096 B2

Jouve, 18, rue Saint-Denis, 75001 PARIS

## Description

Technical field

The present invention relates to a tube set intended for extracorporeal treatment of blood and similar perishable liquids, e.g. by dialysis, comprising at least one pump segment adapted so as to be introduced into a tube pump or the like on a control unit and provided with an inlet end and an outlet end.

Such tube sets normally are made to be used only once. They are frequently divided into an arterial part and a venous part, the arterial part being intended to be coupled between one of the patient's arteries and the apparatus by means of which the blood is to be treated. The venous part is intended instead to be coupled between the said apparatus and one of the patient's veins for the return of the blood. The expression tube sets in the foregoing and in the following refers first and foremost to the arterial part. However, the invention may also be applied to a venous part or to a tube set consisting of combinations of an arterial part and a venous part.

Background art

Present tube sets normally consist of a number of tubes which are joined to one another by a number of branch pipes, T-pipes or other components such as pump segments, drip chambers, pressure monitoring pads or the like, by means of which the flow medium can be controlled and/ or guided. At the same time the tube set normally includes different types of ports for the feed and/or withdrawal of liquids, e.g. for dilution and/or sampling. The said components are usually distributed at different points along the tube set.

A tube set of the above kind is described in for instance DE-A-2 740 062. In said publication the ends of two pump segments are shown fixed in a tensioning member belonging to a control unit in the form of a suitcase.

The present invention primarily has the object of.facilitating the assembling of the tube set on the front of a control unit, the abovementioned components and functions associated therewith being concentrated in one or a small number of points in particular on the front of this control unit. If possible, therefore, the tube set should consist of an unbroken tube between the front of this control unit and the patient. In this manner the personnel performing the treatment can concentrate on the control unit and on the patient. The invention is also intended to facilitate sampling and other treatment of the patient.

Disclosure of the invention

The invention thus relates to a tube set intended for extracorporeal treatment of blood and similar perishable liquids, e.g. by dialysis comprising at least one pump segment adapted so as to be introduced into a tube pump or the like on a control unit and provided with an inlet end and an outlet end. The invention is characterized in that the said inlet end and outlet end respectively are rigidly connected with one another by a spacer formed as a part of the tube set and attachable together with the rest of the tube set to the front of said control unit.

In this manner the assembling of the pump segment is facilitated, at the same time as wrong assembly of the same is prevented.

The said spacer preferably comprises four tube seats, two of which are intended for the pump segment and the remaining two for an inlet part and outlet part respectively of the tube set. This facilitates the assembly of the tube set itself.

In a preferred embodiment of the invention the inlet part which is intended to be connected to an artery moreover comprises an arterial pressure monitor in direct connection with the spacer. Normally such an arterial pressure monitor consists of a plastic pad or the like whose internal pressure can be measured either from the outside or through any suitable connecting nozzle. By joining the arterial pressure monitor directly with the spacer a more compact construction is obtained at the same time as the number of coupling points is reduced.

The spacer may comprise, moreover, one or more ports or the like for the feed and/or withdrawal of material, e.g. for dilution and/or sampling. Such ports or the like are normally arranged in branch pipes or T-pieces, and these can be omitted, therefore, if the ports are provided directly in the spacer.

The spacer preferably comprises, moreover, between the outlet end of the pump segment and the outlet part of the tube set a pipe fitting connecting them. This pipe fitting is preferably adapted so as to comprise an inlet for the input of a reagent, e.g. heparin, and a port for sampling arranged upstream of this inlet. The distance between these components in respect of the outlet end of the pump segment is appropriately chosen so that sampling can take place without any risk of interference by the reagent introduced through back suction from the pump segment.

In a preferred embodiment of the object of the invention the spacer comprises a stay intended to support it against the pump or any part firmly attached to the same during the assembling of the pump segment in the pump or during sampling. This facilitates the said assembling as well as any sampling through ports provided in the spacer. The spacer may comprise also between the inlet part of the tube set and the inlet end of the pump segment a pipe fitting connecting them. This pipe fitting may comprise, furthermore, a port for the input of a liquid, e.g. priming liquid, with the help of the pump.

Brief description of drawings

Fig. 1 shows schematically a dialyser together with a control unit intended for the same and a tube set joining together these components and a patient consisting of an arterial part and a venous part.

Fig. 2 and 3 show two views at right angles to one another of a spacer included in the tube set. Fig. 2 is shown partly in section.

Fig. 4 and 5 finally show sections of the spacer according to fig. 2.

Best mode of carrying out the invention

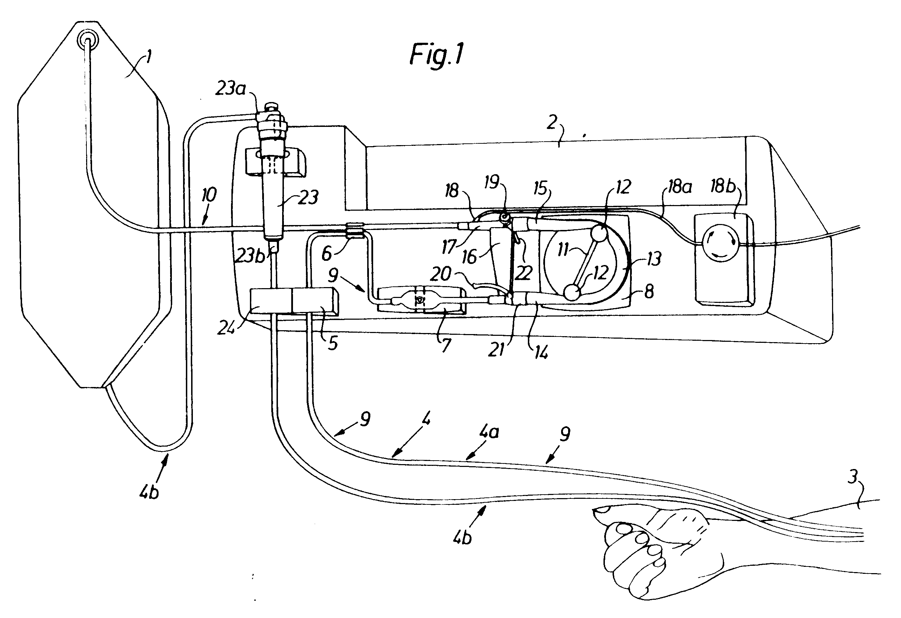

Fig. 1 thus shows a dialyser 1, a control unit 2 and a patient 3 coupled together by means of a tube set designated as a whole by 4. This tube set consists of an arterial part 4a and a venous part 4b. The arterial part 4a is connected by means of an injection needle, not shown, to one of the arteries of the patient 3 and passes through a shut-off clip 5, a holder 6 and an arterial pressure monitor 7 to a pump 8. The part extending to the suction side of the pump constitutes the inlet part of the arterial set and has been given as a whole the designation 9. The part between the delivery side of the pump and the dialyser 1 constitutes the outlet part of the arterial set and has been given as a whole the designation 10.

Since the invention resides first and foremost in the tube set shown, the control unit 2 has been shown only schematically without the instruments and many other components which are normally included in such a control unit. The pump 8 comprises in addition to the rotor 11 with its two rollers 12, firmly installed in the control unit, a pump segment 13 incorporated in the tube set. The inlet end 14 and the outlet end 15 of this pump segment 13 are rigidly connected with one another via a spacer 16. This spacer comprises between the outlet end 15 of the pump segment and the outer part 10 of the tube set a pipe fitting 17 connecting them. This pipe fitting 17 in turn comprises an inlet 18 for the input of a reagent, e.g. heparin, and a port 19 for sampling arranged upstream of this inlet. The distances between these components in respect of the outlet end 15 of the pump segment are chosen so that sampling can take place without any risk of interference by the reagent introduced through back suction from the pump segment 13. A source of heparin or the like is connected to the port or inlet nozzle 18 by a tube 18a via a pump 18b.

In the same manner the spacer 16 is provided between the inlet part 9 and the inlet end 14 of the pump segment with a pipe fitting 21 connecting the same. This pipe fitting too comprises a port, in this case in the form of a closed tube end 20, for the input of a liquid, e.g. priming liquid, with the help of the pump 8. Finally, the spacer comprises a stay 22 intended to support it against the front of the control units or against any other component firmly attached to the pump 8.

From the dialyser 1 the venous part 4b of the tube set passes via a drip chamber 23 with an inlet 23a and an outlet 23b and via a shut-off clip 24 back to the patient 3.

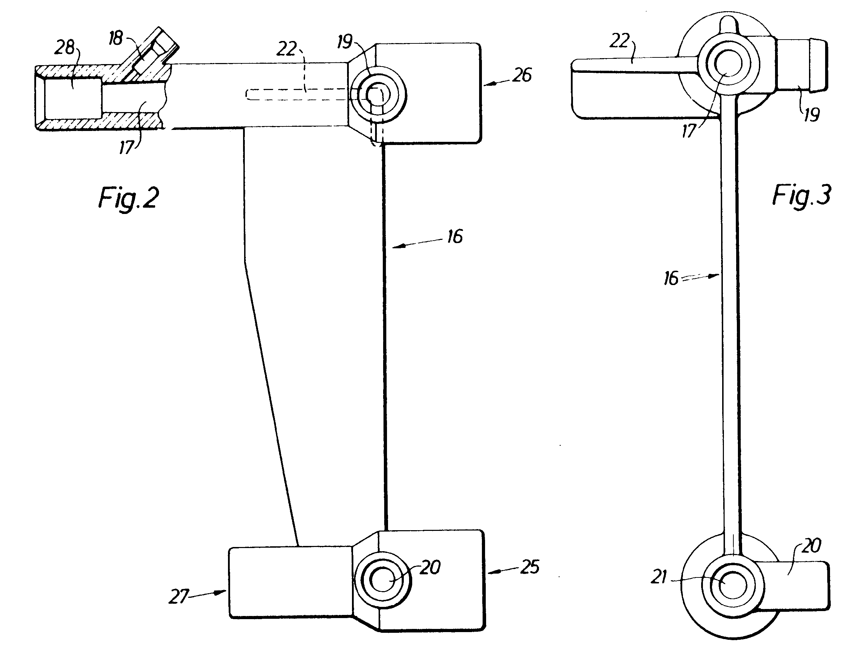

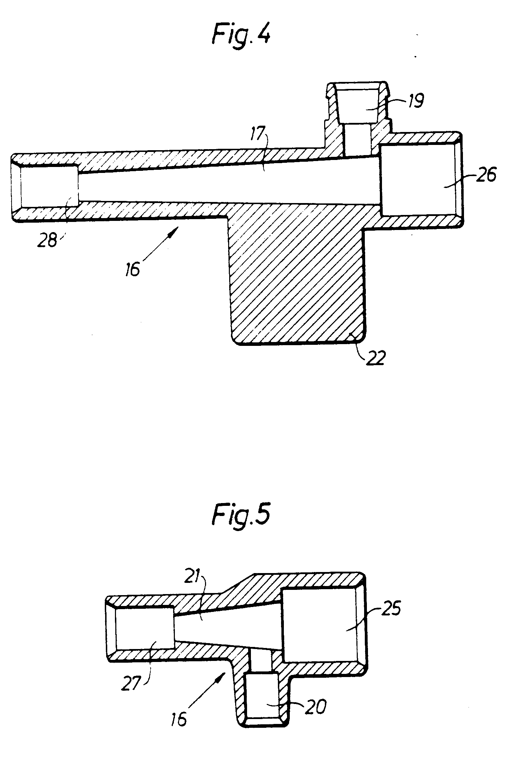

Fig. 2-5 show the spacer 16 in greater detail. As the same reference numerals have been used in these figures as in fig. 1 no further description ought to be necessary. However, the seats for the inlet end 14 and outlet end 15 of the pump segment are designated 25 and 26 respectively. Moreover, the spacer 16 is provided with two further seats 27 and 28, the first of which is intended to be coupled together directly with the outlet end of the arterial pressure monitor 7 terminating the inlet part 9 of the tube set, and the other with the outlet part 10 of the tube set.

Naturally the invention is not limited simply to the preferred embodiment described above but can be varied within the scope of the claims. The different ports mentioned above, for example, may be arranged so as to fulfil functions other than those mentioned by way of example. Furthermore, the tube set can be provided, of course, with further components so as to perform other functions.

**Claims**

1. A tube set intended for extracorporeal treatment of blood and similar perishable liquids, i e by dialysis, comprising at least one pump segment (13) adapted so as to be introduced into a tube pump (8) or the like on a control unit (2) and provided with an inlet end (14) and outlet end (15), said inlet end (14) and outlet end (15) respectively being rigidly connected with one another by a spacer (16) formed as a part of the tube set (4) and attachable together with the rest of the tube set to the front of the control unit (2), said spacer (16) comprising four tube seats (25-28) two of which (25,26) are intended for the pump segment (13) and the remaining two (27,28) for an inlet part (9) and outlet part (10) respectively of the tube set (4), **characterized** in that the spacer (16) comprises one or more ports (19,20) for the feed and/or withdrawal of material, e g for dilution and/or sampling.

2. A tube set in accordance with claim 1, **characterized** in that its inlet part (9) which is intended to be connected to an artery moreover comprises an arterial pressure monitor (7) in direct connection with the spacer (16).

3. A tube set in accordance with claim 1, **characterized** in that the spacer (16) comprises, between the outlet end (15) of the pump segment (13) and the outlet part (10) of the tube set (4), a pipe fitting (17) connecting them.

4. A tube set in accordance with claim 3, **characterized** in that the said pipe fitting (17) comprises an inlet (18) for the input of a reagent, e g heparin, and a port (19) arranged upstream of this inlet for sampling, the distance betwen these components in respect of the outlet end (15) of the pump segment (13) being such that sampling can take place without any risk for interference by the reagent introduced through back suction from the pump segment.

5. A tube set in accordance with anyone of the preceding claims, **characterized** in that the spacer (16) comprises a stay (22) intended to support it against the pump (8) or any part firmly attached to the same during the assembling of the pump segment (13) in the pump or during sampling.

6. A tube set in accordance with claim 1, **characterized** in that the spacer (16) comprises between the inlet part (9) of the tube set (4) and the inlet end (14) of the pump segment (13) a pipe fitting (21) connecting them.

7. A tube set in accordance with claim 6, **characterized** in that the said pipe fitting (21) comprises a port (20) for the input of a liquid, e g priming liquid, with the help of the pump (8).

**Patentansprüche**

1. Schlauchgarnitur für die Behandlung von Blut und ähnlichen verderblichen Flüssigkeiten außerhalb des Körpers, d. h. durch Dialyse, mit mindestens einem Pumpensegment (13), welches geeignet derart ausgestaltet ist, daß es in eine Schlauchpumpe (8) oder dergleichen auf einer Steuereinheit (2) eingeführt wird, und mit einem Einlaßende (14) und einem Auslaßende (15) versehen ist, wobei das Einlaßende (14) und das Auslaßende (15) jeweils durch ein Abstandsstück (16) star miteinander verbunden sind, welches als Teil der Schlauchgarnitur (4) gebildet ist und zusammen mit der übrigen Schlauchgarnitur an der Vorderseite der Steuereinhert (2) anbringbar ist, und wobei das Abstandsstück (16) vier Schlauchaufnahmen (25 - 28) aufweist, von denen zwei (25, 26) für das Pumpensegment (13) und die übrigen zwei (27, 28) für ein Einlaßteil (9) bzw. Auslaßteil (10) der Schlauchgarnitur (4) gedacht sind, **dadurch gekennzeichnet**, daß das Abstandsstück (16) eine oder mehrere Öffnungen (19, 20) für die zufuhr und/oder das Abziehen von Material, z. B. für Verdünnung und-/oder Probenahme, aufweist.

2. Schlauchgarnitur nach Anspruch 1, **dadurch gekennzeichnet**, daß ihr Einlaßteil (9), das mit einer Arterie verbunden werden soll, ferner einen Arteriendruckmonitor (7) in direkter Verbindung mit dem Abstandsstück (16) aufweist.

3. Schlauchgamitur nach Anspruch 1, **dadurch gekennzeichnet**, daß das Abstandsstück (16) zwischen dem Auslaßende (15) des Pumpensegmentes (13) und dem Auslaßteil (10) der Schlauchgamitur (4) ein sie verbindendes Leitungsanschlußstück (17) aufweist.

4. Schlauchgarnitur nach Anspruch 3, **dadurch gekennzeichnet**, daß das Leitungsanschlußstück (17) einen Einlaß (18) für den Eingang eines Reagens, z. B. Heparin, und eine Öffnung (19) aufweist, die aufstromseitig von diesem Einlaß für die Probenahme angeordnet ist, wobei der Abstand zwischen diesen Bestandteilen bezüglich des Auslaßendes (15) des Pumpensegmentes (13) derart ist, daß die Probenahme ohne irgendein Risiko der Störung durch das Reagens erfolgen kann, welches durch die Rückeinsaugung aus dem Pumpensegment eingeführt ist.

5. Schlauchgarnitur nach einem der vorhergehenden Ansprüche, **dadurch gekennzeichnet**, daß das Abstandsstück (16) eine Stütze (22) aufweist, die für seine Abstützung gegen die Pumpe (8) oder irgendein Teil vorgesehen ist, welches fest an derselben angebracht ist, und zwar während der Anordnung des Pumpensegmentes (13) in der Pumpe oder während der Probenahme.

6. Schlauchgarnitur nach Anspruch 1, **dadurch gekennzeichnet**, daß das Abstandsstück (16) zwischen dem Einlaßteil (9) der Schlauchganitur (4) und dem Einlaßende (14) des Pumpensegmentes (13) ein sie verbindendes Leitungsanschlußstück (21) aufweist.

7. Schlauchgarnitur nach Anspruch 6, **dadurch gekennzeichnet**, daß das Leitungsanschlußstück (21) eine Öffnung (20) aufweist für den Einlaß einer Flüssigkeit, z. B. Anlaßflüssigkeit, mit Hilfe der Pumpe (8).

**Revendications**

1. Tubulure pour le traitement extracorporel du sang et des liquides périssables analogues, par exemple par dialyse, comprenant au moins un segment de pompe (13) conçu pour être introduit dans une pompe péristaltique ou analogue (8) montée sur une unité de commande (2) et comportant une extrémité d'entrée (14) et une extrémité de sortie (15), ladite extrémité d'entrée (14) et ladite extrémité de sortie (15) respectivement étant rigidement reliées l'une à l'autre par un écarteur (16) formé en tant que partie de la tubulure (4) et fixable avec le reste de la tubulure sur la face avant de l'unité de commande (2), ledit écarteur (16) comprenant quatre sièges de tube (25-28) dont deux (25,26) sont destinés au segment de pompe (13) et dont les deux sièges restants (27,28) sont destinés respectivement à une partie amont (9) et à une partie aval (10) de la tubulure (4), caractérisée en ce que l'écarteur (16) comprend un ou plusieurs orifices (19,20) pour l'administration et/ou le prélèvement de substances, par exemple pour dilution et/ou prélèvement d'échantillons.

2. Tubulure suivant la revendication 1, caractérisée en ce que sa partie amont (9), qui est destinée à être reliée à une artère, comprend en outre un dispositif de contrôle de pression artérielle (7) en liaison directe avec l'écarteur (16).

3. Tubulure suivant la revendication 1, caractérisée en ce que l'écarteur (16) comprend, entre l'extrémité de sortie (15) du segment de pompe (13) et la partie aval (10) de la tubulure (4), un raccord (17) reliant celles-ci.

4. Tubulure suivant la revendication 3, caractérisée en ce que ledit raccord (17) comprend une entrée (18) pour l'introduction d'un réactif, tel que l'héparine, et un orifice (19) placé en amont de cette entrée pour le prélèvement d'échantillons, la distance entre ces éléments par rapport à l'extrémité de sortie (15) du segment de pompe (13) étant telle que le prélèvement d'échantillons peut s'effectuer sans aucun risque d'interférence avec le réactif introduit par rétro-aspiration du segment de pompe.

5. Tubulure suivant l'une quelconque des revendications précédentes, caractérisée en ce que l'écarteur (16) comprend un support (22) destiné à le faire porter contre la pompe (8) ou toute partie solidement fixée à la pompe lors du montage du segment de pompe (13) dans la pompe ou lors du prélèvement d'échantillons.

6. Tubulure suivant la revendication 1, caractérisée en ce que l'écarteur (16) comprend, entre la partie amont (9) de la tubulure (4) et l'extrémité d'entrée (14) du segment de pompe (13), un raccord de tuyau (21) qui relie celles-ci.

7. Tubulure suivant la revendication 6, caractérisée en ce que ledit raccord de tuyau (21) comprend un orifice d'accès (20) pour l'admission d'un liquide, tel qu'un liquide d'amorçage, au moyen de la pompe (8).

Fig.1

Fig.3

Fig.2

## Fig.4

## Fig.5